(19) Europäisches Patentamt
European Patent Office
Office européen des brevets

(11) Veröffentlichungsnummer: **0 086 901**
**B1**

(12) # EUROPÄISCHE PATENTSCHRIFT

(45) Veröffentlichungstag der Patentschrift: **04.12.85**

(51) Int. Cl.⁴: **C 07 D 233/60**, C 07 D 249/08, A 01 N 43/653, A 01 N 43/50

(21) Anmeldenummer: **82112016.9**

(22) Anmeldetag: **27.12.82**

(54) **Azolyl-thioether-Derivate, Verfahren zu ihrer Herstellung sowie ihre Verwendung als Fungizide und Pflanzenwachstumsregulatoren.**

(30) Priorität: **09.01.82 DE 3200414**

(43) Veröffentlichungstag der Anmeldung: **31.08.83 Patentblatt 83/35**

(45) Bekanntmachung des Hinweises auf die Patenterteilung: **04.12.85 Patentblatt 85/49**

(84) Benannte Vertragsstaaten: **AT BE CH DE FR GB IT LI NL**

(56) Entgegenhaltungen: **EP-A-0 059 894**
**DE-A-2 105 490**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Regel, Erik, Ing., grad., Untere Bergerheide 26, D-5600 Wuppertal 1 (DE)**
Erfinder: **Draber, Wilfried, Dr., In den Birken 81, D-5600 Wuppertal 1 (DE)**
Erfinder: **Büchel, Karl Heinz, Prof. Dr., Dabringhausener Strasse 42, D-5093 Burscheid (DE)**
Erfinder: **Frohberger, Paul-Ernst, Dr., Willi-Baumeister-Strasse 5, D-5090 Leverkusen (DE)**
Erfinder: **Lürssen, Klaus, Dr., August-Kierspel-Strasse 89, D-5070 Bergisch-Gladbach 2 (DE)**

EP 0 086 901 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft neue Azolyl-thioether-Derivate, mehrere Verfahren zu ihrer Herstellung sowie ihre Verwendung als Fungizide und Pflanzenwachstumsregulatoren.

Es ist bereits bekannt geworden, daß bestimmte 1-Halogen-2,2-dimethyl-5-phenyl-4-triazolyl-3-pentanone, wie z. B. 2,2-Dimethyl-1-fluor-5-(4-methylphenyl)-4-(1,2,4-triazol-1-yl)-3-pentanon, 2,2-Dimethyl-1-fluor-5-phenyl-4-(1,2,4-triazol-1-yl)-3-pentanol, 2,2-Dimethyl-1-fluor-5-(2,4-dichlorphenyl)-4-(1,2,4-triazol-1-yl)-3-pentanon und 1-Chlor-2,2-dimethyl-5-phenyl-4-(1,2,4-triazol-1-yl)-3-pentanol, gute fungizide und pflanzenwachstumsregulierende Eigenschaften aufweisen (vergleiche DE-OS 2 951 164 und DE-OS 2 951 163). Die Wirkung dieser Verbindungen ist jedoch, insbesondere bei niedrigen Aufwandmengen und -konzentrationen, nicht immer voll befriedigend.

Es wurden neue Azolyl-thioether-Derivate der Formel

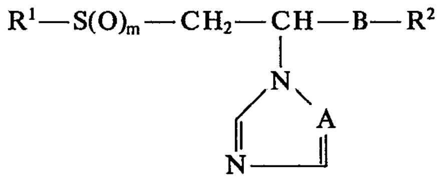

(I)

in welcher

A für ein Stickstoffatom oder die CH-Gruppe steht,
B für die CO- oder CH(OH)-Gruppe steht,
m für die Zahlen 0 oder 2 steht,
$R^1$ für gegebenenfalls einfach oder mehrfach, gleich oder verschieden substituiertes Phenyl steht, wobei als Substituenten Halogen, Alkyl mit 1 bis 4 Kohlenstoffatomen, Halogenalkyl, Halogenalkoxy und Halogenalkylthio mit jeweils 1 bis 2 Kohlenstoff- und 1 bis 5 gleichen oder verschiedenen Halogenatomen, Nitro, Cyano und gegebenenfalls durch Halogen oder Alkyl mit 1 bis 2 Kohlenstoffatomen substituiertes Phenyl zu nennen sind,
$R^2$ für gegebenenfalls durch Alkyl mit 1 bis 4 Kohlenstoffatomen substituiertes Cycloalkyl mit 3 bis 7 Kohlenstoffatomen, sowie für die Gruppierungen

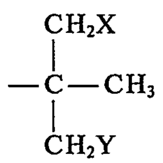 und 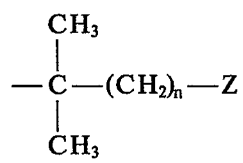

steht, wobei

X für Wasserstoff, Fluor oder Chlor steht,
Y für Wasserstoff, Fluor oder Chlor steht,
Z für Alkyl mit 1 bis 4 Kohlenstoffatomen, für jeweils gegebenenfalls substituiertes Phenyl, Phenoxy, Phenylthio, Benzyloxy oder Benzylthio steht, wobei als Phenylsubstituenten die bei $R^1$ erwähnten Phenylsubstituenten zu nennen sind, ferner für Alkoxy und Alkylthio mit jeweils 1 bis 4 Kohlenstoffatomen, für Halogenalkoxy und Halogenalkylthio mit jeweils 1 bis 2 Kohlenstoff- und 1 bis 5 gleichen oder verschiedenen Halogenatomen steht, sowie für Alkoxycarbonyl mit 1 bis 4 Kohlenstoffatomen im Alkylteil und für Cyano steht, und
n für die Zahlen 0 oder 1 steht,

sowie deren Säureadditions-Salze und Metallsalz-Komplexe gefunden.

Die Verbindungen der Formel (I) besitzen für den Fall, daß B für CH(OH) steht, zwei asymmetrische Kohlenstoffatome; sie können dann in der erythro- wie in der threo-Form vorliegen. Im allgemeinen fallen sie als Diastereomerengemische unterschiedlicher Zusammensetzung an. In allen Fällen liegen sie vorwiegend als Racemate vor.

Weiterhin wurde gefunden, daß man die Azolyl-thioether-Derivate der Formel (I) sowie deren Säureadditions-Salze und Metallsalz-Komplexe erhält, wenn man

a) Azolylketone der Formel

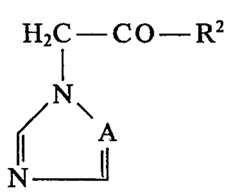

(II)

in welcher
A und $R^2$ die oben angegebene Bedeutung haben,
mit Formaldehyd oder Formaldehyd abgebenden Stoffen, wie Paraformaldehyd, und einem Thio-Derivat der Formel

$$R^1—S—H \qquad (III)$$

in welcher
$R^1$ die oben angegebene Bedeutung hat,
in Gegenwart eines Verdünnungsmittels und in Gegenwart eines Katalysators umsetzt; und gegebenenfalls
b) die so erhaltenen Verbindungen der Formel

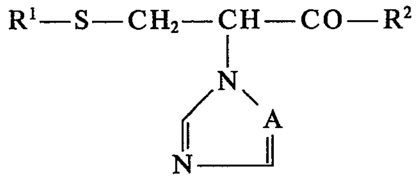

(Ia)

in welcher
A, $R^1$ und $R^2$ die oben angegebene Bedeutung haben,
nach bekannten Methoden in üblicher Weise zu den entsprechenden $SO_2$-Derivaten oxidiert, und gegebenenfalls
c) die nach den Verfahren (a) und (b) erhaltenen Verbindungen der Formel

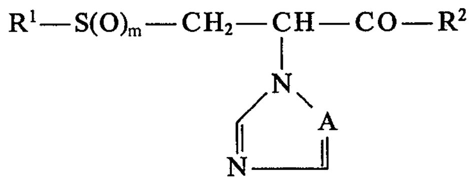

(Ib)

in welcher
A, $R^1$, $R^2$ und m die oben angegebene Bedeutung haben,
nach bekannten Methoden in üblicher Weise zu den entsprechenden CH(OH)-Derivaten reduziert; und gegebenenfalls anschließend an die nach den Verfahren (a), (b) und (c) erhaltenen Verbindungen der Formel (I) in üblicher Weise eine Säure oder ein Metallsalz addiert.

Schließlich wurde gefunden, daß die neuen Azolyl-thioether-Derivate der Formel (I) starke fungizide und pflanzenwachstumsregulierende Eigenschaften aufweisen. Dabei zeigen überraschenderweise die erfindungsgemäßen Verbindungen der Formel (I) eine bessere Wirkung als die aus dem Stand der Technik bekannten Verbindungen 2,2-Dimethyl-1-fluor-5-(4-methylphenyl)-4-(1,2,4-triazol-1-yl)-3-pentanon, 2,2-Dimethyl-1-fluor-5-phenyl-4-(1,2,4-triazol-1-yl)-3-pentanol, 2,2-Dimethyl-1-fluor-5-(2,4-dichlorphenyl)-4-(1,2,4-triazol-1-yl)-3-pentanon und 1-Chlor-2,2-dimethyl-5-phenyl-4-(1,2,4-triazol-1-yl)-3-pentanol, welche chemisch und wirkungsmäßig ähnliche Verbindungen sind. Die erfindungsgemäßen Stoffe stellen somit eine Bereicherung der Technik dar.

Die erfindungsgemäßen Azolyl-thioether-Derivate sind durch die Formel (I) allgemein definiert.

Bevorzugt sind diejenigen Verbindungen der Formel (I), in denen

$R^1$ für gegebenenfalls einfach bis dreifach, gleich oder verschieden substituiertes Phenyl steht, wobei als Substituenten genannt seien: Fluor, Chlor, Methyl, Ethyl, Isopropyl, tert.-Butyl, Trifluormethyl, Trifluormethoxy, Trifluormethylthio, Nitro, Cyano, sowie gegebenenfalls durch Fluor, Chlor und/oder Methyl substituiertes Phenyl;
$R^2$ für gegebenenfalls durch Methyl, Ethyl oder Propyl substituiertes Cyclopropyl, Cyclopentyl und Cyclohexyl steht, sowie für die Gruppierungen

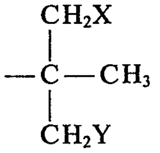 und 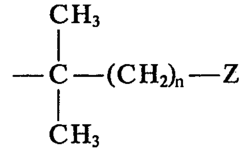

steht, wobei

X für Wasserstoff, Fluor oder Chlor steht,

Y für Wasserstoff, Fluor oder Chlor steht,
Z für Alkyl, Alkoxy und Alkylthio mit jeweils 1 bis 4 Kohlenstoffatomen, für Trifluormethoxy und Trifluormethylthio, für Methoxy-, Ethoxy- und Isopropoxycarbonyl sowie für Cyano steht, ferner für gegebenenfalls einfach bis dreifach substituiertes Phenyl, Phenoxy oder Phenylthio steht, wobei als Substituenten die bei $R^1$ bereits genannten Phenylsubstituenten infrage kommen; und
A, B, m und n für die in der Erfindungsdefinition angegebenen Bedeutungen stehen.

Bevorzugte erfgindungsgemäße Verbindungen sind auch Additionsprodukte aus Säuren und denjenigen Azolyl-thioether-Derivaten der Formel (I), in denen die Reste A, B, $R^1$ und $R^2$ sowie der Index m die Bedeutungen haben, die bereits vorzugsweise für diese Reste und den Index genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, sowie Sulfonsäuren, wie z.B. p-Toluolsulfonsäure und 1,5-Naphthalindisulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe und denjenigen Azolyl-thioether-Derivaten der Formel (I), in denen die Reste A, B, $R^1$ und $R^2$ sowie der Index m die Bedeutungen haben, die bereits vorzugsweise für diese Reste und en Index genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Verwendet man beispielsweise 1,2,4-Triazol-pinakolin, Paraformaldehyd und 4-Chlorthiophenol als Ausgangsstoffe, so kann der Reaktionsablauf durch das folgende Formelschema wiedergegeben werden (Verfahren a):

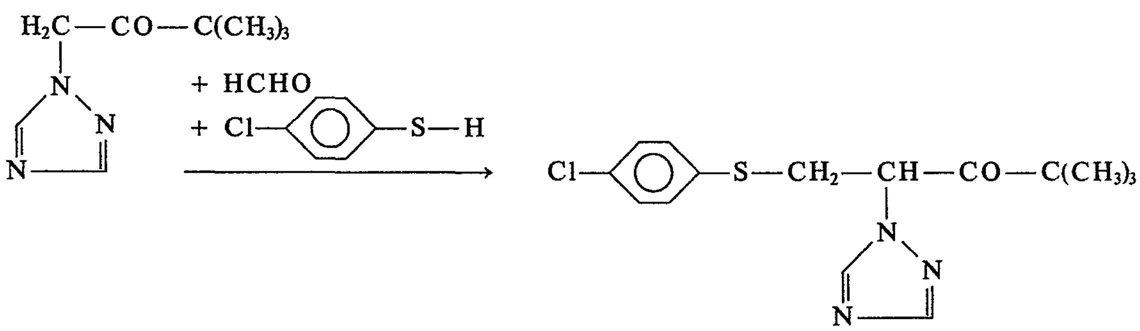

Verwendet man beispielsweise 1-(4-Chlorphenylthio)-4,4-dimethyl-2-(1,2,4-triazol-1-yl)-3-pentanon und Wasserstoffperoxid in Eisessig als Ausgangsstoffe, so kann der Reaktionsablauf durch das folgende Formelschema wiedergegeben werden (Verfahren b):

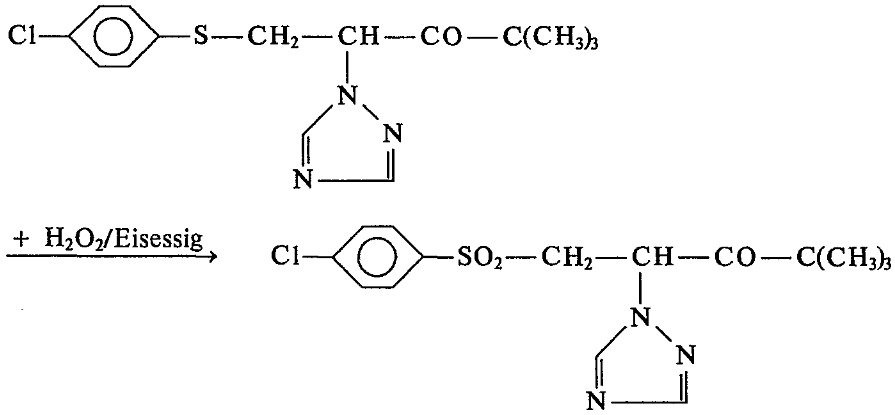


Verwendet man beispielsweise 1-(4-Chlorphenylthio)-4,4-dimethyl-2-(1,2,4-triazol-1-yl)-3-pentanon und Natriumborhydrid als Ausgangsstoffe, so kann der Reaktionsablauf durch das folgende Formelschema wiedergegeben werden (Verfahren c):

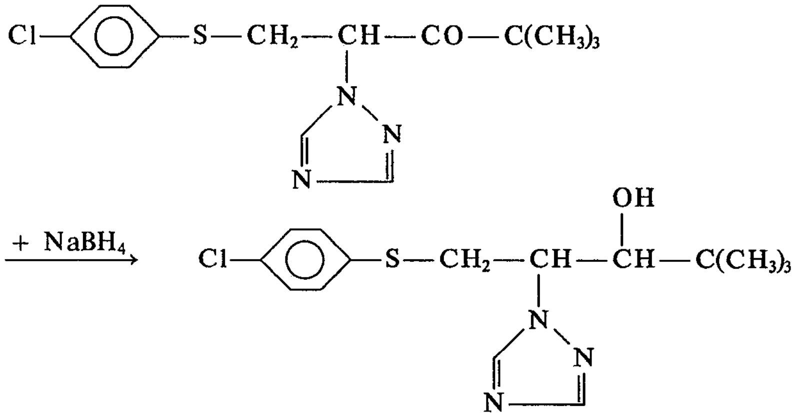


Die für das erfindungsgemäße Verfahren (a) als Ausgangsstoffe zu verwendenden Azolyl-Ketone sind durch die Formel (II) allgemein definiert. In dieser Formel stehen A und $R^2$ vorzugsweise für diejenigen Bedeutungen, die bereits im Zusammenhang mit der Beschreibung der erfindungsgemäßen Stoffe der Formel (I) vorzugsweise für diese Subsituenten genannt wurden.

Die Azolyl-ketone der Formel (II) sind bekannt (vergleiche z.B. DE-OS 24 31 407, DE-OS 26 38 470, DE-OS 28 20 361 und DE-OS 30 10 560), bzw. sind sie Gegenstand eigener älterer Patentanmeldungen, die noch nicht veröffentlicht sind (vergleiche die Deutschen Patentanmeldungen P 30 28 330 vom 25.7.1980 und P 30 48 266 vom 20.12.1980, bzw. können sie in allgemein bekannter Art und Weise erhalten werden, indem man entsprechende Halogenketone mit 1,2,4-Triazol oder Imidazol in Gegenwart eines inerten organischen Lösungsmittels, wie beispielsweise Aceton, und in Gegenwart eines Säurebindemittels, wie beispielsweise Kaliumcarbonat, vorzugsweise in der Siedehitze des verwendeten Lösungsmittels umsetzt.

Die außerdem für das erfindungsgemäße Verfahren (a) als Ausgangsstoffe zu verwendenden Thio-Derivate sind durch die Formel (III) allgemein definiert. In dieser Formel steht $R^1$ vorzugsweise für diejenigen Bedeutungen, die bereits im Zusammenhang mit der Beschreibung der erfindungsgemäßen Stoffe der Formel (I) vorzugsweise für diesen Substituenten genannt wurden.

Die Thio-Derivate der Formel (III) sind allgemein bekannte Verbindungen der organischen Chemie.

Als Lösungsmittel für das erfindungsgemäße Verfahren (a) kommen vorzugsweise unter den Reaktionsbedingungen inerte organische Lösungsmittel infrage. Hierzu gehören vorzugsweise Alkohole, wie Methanol und Ethanol; Ether, wie Tetrahydrofuran und Dioxan; aliphatische und cycloaliphatische Kohlenstoffwasserstoffe, wie Hexan und Cyclohexan; aromatische Kohlenwasserstoffe, wie Benzol, Toluol und Xylol; halogenierte aliphatische und aromatische Kohlenwasserstoffe, wie Methylenchlorid und Tetrachlorkohlenstoff, Chloroform, Chlorbenzol und Dichlorbenzol.

Das erfindungsgemäße Verfahren (a) wird in Gegenwart eines Katalysators durchgeführt. Man kann alle üblicherweise verwendbaren sauren und insbesondere basischen Katalysatoren sowie deren Puffergemische einsetzen. Hierzu gehören vorzugsweise Lewis-Säuren, wie z.B. Bortrifluorid, Bortrichlorid, Zinntetrachlorid oder Titantetrachlorid; organische Basen, wie Pyridin und Piperidin; sowie insbesondere Piperidinacetat.

Die Reaktionstemperaturen können bei der Durchführung des erfindungsgemäßen Verfahrens (a) in einem größeren Bereich variiert werden. Im allgemeinen arbeitet man zwischen 20 und 160°C, vorzugsweise bei der Siedetemperatur des jeweiligen Lösungsmittels.

Bei der Durchführung des erfindungsgemäßen Verfahrens (a) arbeitet man vorzugsweise in äquimolaren Mengen, wobei geringe Über- oder Unterschreitungen der einzelnen Reaktionspartner möglich sind. Die Isolierung der Verbindungen der Formel (I) erfolgt in üblicher Weise.

Die erfindungsgemäße Oxidation gemäß Verfahren (b) erfolgt durch die Umsetzung mit üblichen anorganischen oder organischen Oxidationsmitteln. Hierzu gehören vorzugsweise organische Persäuren, wie z.B. Peressigsäure, p-Nitroperbenzoesäure, m-Chlorperbenzoesäure; anorganische Persäuren, wie z.B. Periodsäure; weiterhin Wasserstoffperoxid in Eisessig oder Methanol, Kaliumpermanganat und Chromsäure.

Die Reaktionstemperaturen können bei der Durchführung der Oxidation in einem größeren Bereich variiert werden. Im allgemeinen arbeitet man zwischen etwa −50 bis 100°C, vorzugsweise zwischen 10 und 80°C.

Bei der Durchführung der erfindungsgemäßen Oxidation setzt man auf 1 Mol der erfindungsgemäßen Verbindungen der Formel (Ia) etwa 1 bis 5 Mol Oxidationsmittel, wie m-Chlorperbenzoesäure in Methylenchlorid oder Wasserstoffperoxid in Acetanhydrid bzw. Eisessig ein. Die Isolierung der Oxidationsprodukte erfolgt in üblicher Weise. In einer besonderen Durchführungsform der erfindungsgemäßen Oxidation verwendet man überschüssige Mengen Wasserstoffperoxid in Gegenwart von Titan(III)-chlorid-Lösung bei Raumtemperatur (vergleiche hierzu auch Synthesis Communications, S 205ff (1981)). Überraschenderweise entstehen hierbei nicht, wie in der genannten Literaturstelle beschrieben, die SO-Derivate, sondern die entsprechenden $SO_2$-Derivate.

Die erfindungsgemäße Reduktion gemäß Verfahren (c) erfolgt in üblicher Weise, wie z.B. durch Umsetzung mit komplexen Hydriden, gegebenenfalls in Gegenwart eines Verdünnungsmittels, oder durch Umsetzung mit Aluminiumisopropylat in Gegenwart eines Verdünnungsmittels.

Arbeitet man mit komplexen Hydriden, so kommen als Verdünnungsmittel für die erfindungsgemäße Umsetzung polare organische Lösungsmittel infrage. Hierzu gehören vorzugsweise Alkohole, wie Methanol, Ethanol, Buthanol, Isopropanol, und Ether, wie Diethylether oder Tetrahydrofuran. Die Reaktion wird im allgemeinen bei −15 bis 30°C, vorzugsweise bei −15 bis +20°C durchgeführt. Hierzu setzt man auf 1 Mol des Ketons der Formel (1 b) etwa 1 Mol eines komplexen Hydrids, wie Natriumborhydrid, Calciumborhydrid oder Lithiumalanat, ein. Zur Isolierung der reduzierten Verbindungen der Formel (I) wird der Rückstand in verdünnter Salzsäure aufgenommen, anschließend alkalisch gestellt und mit einem organischen Lösungsmittel extrahiert. Die weitere Aufarbeitung erfolgt in üblicher Weise.

Arbeitet man mit Aluminiumisopropylat, so kommen als Verdünnungsmittel für die erfindungsgemäße Umsetzung bevorzugt Alkohole, wie Isopropanol, oder inerte Kohlenwasserstoffe, wie Benzol, infrage. Die Reaktionstemperaturen können wiederum in einem größeren Bereich variiert werden; im allgemeinen arbeitet man zwischen 20 und 120°C, vorzugsweise bei 50 bis 100°C. Zur Durchführung der Reaktion setzt man auf 1 Mol des Ketons der Formel (1 b) etwa 0,3 bis 2 Mol Aluminiumisopropylat ein. Zur Isolierung der reduzierten Verbindungen der Formel (I) wird das überschüssige Lösungsmittel im Vakuum entfernt und die entstandenen Aluminium-Verbindungen mit verdünnter Schwefelsäure oder Natronlauge zersetzt.

Die weitere Aufarbeitung erfolgt in üblicher Weise.

Zur Herstellung von physiologisch verträglichen Säureadditionssalzen der Verbindungen der Formel (I) kommen vorzugsweise folgende Säuren infrage: Die Halogenwasserstoffsäuren, wie z.B. die Chlorwasserstoffsäure und die Bromwasserstoffsäure, insbesondere die Chlorwasserstoffäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Citronensäure, Salicylsäure, Sorbinsäure, Milchsäure, sowie Sulfonsäuren, wie z.B. p-Toluolsulfonsäure und 1,5-Naphthalindisulfonsäure.

Die Säureadditionssalze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I. bis II. sowie IV. bis VIII. Nebengruppe infrage, wobei Kupfer, Zink, Mangan, Magnesium, Zinn, Eisen und Nickel beispielhaft genannt seien. Als Anionen der Salze kommen solche in Betracht, die sich vorzugsweise von folgenden Säuren ableiten: Halogenwasserstoffsäuren, wie z.B. die Chlorwasserstoffsäure und die Bromwasserstoffsäure, ferner Phosphorsäure, Salpetersäure und Schwefelsäure.

Die Metallsalz-Komplexe von Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung solcher Pilze eingesetzt werden, die echte Mehltauerkrankungen hervorrufen, so zur Bekämpfung des Gerstenmehltaus (Erysiphe graminis); sowie zur Bekämpfung von Phytophthora-Arten, wie gegen den Erreger der Braunfäule der Tomate (Phytophthora infestans); und zur Bekämpfung von Reiskrankheiten, wie Pyricularia oryzae und Pellicularia sasakii.

Die erfindungsgemäß verwendbaren Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwachstumsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens (»Lagerns«) der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, sodaß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragssteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, sodaß mehr oder größere Früchte entstehen.

Ertragssteigerungen können in manchen Fällen durch einen Eingriff in den pflanzlichen Stoffwechsel erreicht werden, ohne daß die Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle, ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden (»Ausdünnung«), um die Aternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüberhinaus kann mit Wachstumsregulatoren auch eine zeitliche Konzentrierung der Reife erzielt werden. Damit werden die Voraussetzungen dafür geschaffen, daß z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, sodaß die Pflanzen, wie z.B. Ananas oder Zierpflanzen in Gärtnereien, zu einem Zeitpunkt keimen, austreiben oder blühen, an dem sie normalerweise keine Bereitschaft

zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

In entsprechenden Aufwandmengen und -konzentrationen zeigen die erfindungsgemäßen Stoffe auch eine selektiv herbizide Wirkung.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alokohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummi arabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder der daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Verbindungen als Pflanzenwachstumsregulatoren können die Aufwandmengen in einem größeren Bereich variiert werden. Im allgemeinen verwendet man pro Hektar Bodenfläche 0,01 bis 50 kg, bevorzugt 0,05 bis 10 kg.

Beim Einsatz der erfindungsgemäßen Stoffe als Pflanzenwachstumsregulatoren gilt, daß die Anwendung in einem bevorzugten Zeitraum vorgenommen wird, dessen genaue Abgrenzung sich nach den klimatischen und vegetativen Gegebenheiten richtet.

Auch beim Einsatz der erfindungsgemäßen Stoffe als Fungizide kann die Aufwandmenge je nach Art der Applikation in einem größeren Bereich variiert werden: So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 Gew.-%. Bei der Saatgutbehandlung werden im allgemeinen Wirkstoffmengen von 0,001 bis 50 g je kg Saatgut, vorzugsweise 0,01 bis 10 g, benötigt. Bei Behandlung des Bodens sind Wirkstoffkonzentrationen von 0,0001 bis 0,1 Gew.-%, vorzugsweise von 0,0001 bis 0,02%, am Wirkungsort erforderlich.

## Herstellungsbeispiele

### Beispiel 1

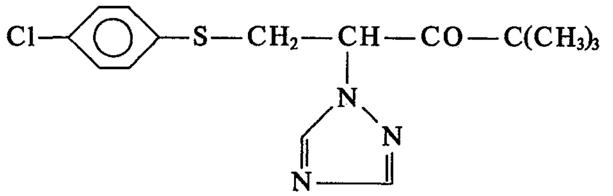

(Verfahren a)

Ein Gemisch aus 72,5 g (0,5 Mol) 4-Chlorthiophenol, 100,2 g (0,6 Mol) 1,2,4-Triazol-1-yl-pinakolin, 18 g (0,6 Mol) Paraformaldehyd, 15 g Essigsäure und 5 ml Piperidin werden in 600 ml Toluol unter Rückfluß am Wasserabscheider erhitzt. Nach beendeter Wasserabscheidung wird die Reaktionslösung mit Wasser gewaschen, über Natriumsulfat getrocknet, filtriert und im Vakuum eingeengt. Der ölige Rückstand wird über eine Kieselgelsäule (Laufmittel: Chloroform) chromatographiert. Man erhält 45 g (28% der Theorie) 1-(4-Chlorphenylthio)-4,4-dimethyl-2-(1,2,4-triazol-1-yl)-3-pentanon vom Schmelzpunkt 67°C.

### Beispiel 2

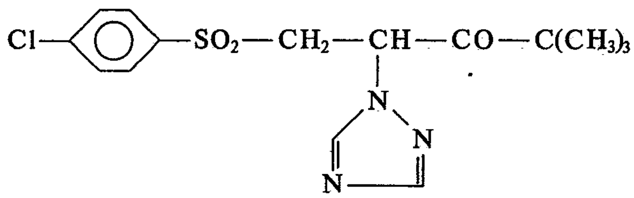

(Verfahren b)

Zu einer Lösung von 22,6 g (0,07 Mol) 1-(4-Chlorphenylthio)-4,4-dimethyl-2-(1,2,4-triazol-1-yl)-3-pentanon (Beispiel 1) in 100 ml Essigsäure und 2 Tropfen konzentrierter Schwefelsäure werden langsam 8,3 g (0,074 Mol) 30%iges Wasserstoffperoxid getropft. Man läßt das Reaktionsgemisch 18 Stunden bei 60°C nachrühren und versetzt es erneut mit 8,3 g (0,074 Mol) 30%igem Wasserstoffperoxid. Das Reaktionsgemisch wird anschließend 18 Stunden bei 80°C nachgerührt. Danach gibt man die Reaktionslösung auf Wasser und extrahiert mit Methylenchlorid. Die organische Phase wird über Natriumsulfat getrocknet, filtriert und im Vakuum eingeengt. Der ölige Rückstand kristallisiert nach Verrühren mit Diisopropylether. Man erhält 4,9 g (22% der Theorie) 1-(4-Chlorphenylsulfonyl)-4,4-dimethyl-2-(1,2,4-triazol-1-yl)-3-pentanon vom Schmelzpunkt 134°C.

### Beispiel 3

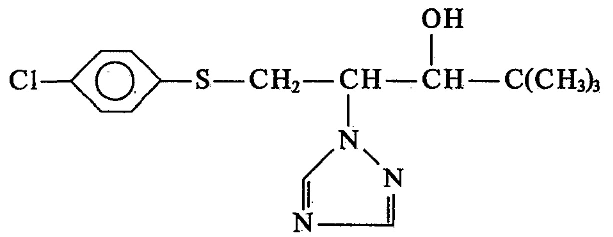

(Verfahren c)

Zu einem Gemisch von 16,2 g (0,05 Mol) 1-(4-Chlorphenylthio)-4,4-dimethyl-2-(1,2,4-triazol-1-yl)-3-pentanon (Beispiel 1) und 3,86 g (0,035 Mol) Calciumchlorid in 250 ml Isopropanol werden bei −15°C langsam 1,39 g (0,0367 Mol) Natriumborhydrid in 30 ml Wasser getropft. Nach 18 Stunden wird das Reaktionsgemisch im Vakuum eingeengt, der Rückstand in Methylenchlorid aufgenommen, mit Wasser gewaschen, über Natriumsulfat getrocknet, filtriert und erneut eingeengt. Der ölige Rückstand kristallisiert nach dem Verreiben mit Diisopropylether. Man erhält 14,3 g (87% der Theorie) 1-(4-Chlorphenylthio)-4,4-dimethyl-2-(1,2,4-triazol-1-yl)-3-pentanol vom Schmelzpunkt 84°C.

Beispiel 4

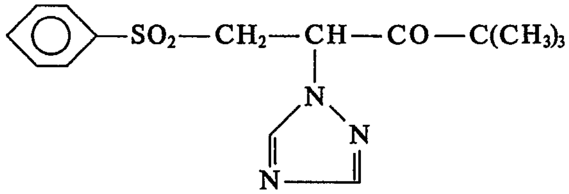

(Verfahren b)

Zu einer Lösung von 5 g (0,0173 Mol) 4,4-Dimethyl-1-phenylthio-2-(1,2,4-Triazol-1-yl)-3-pentanon (erhältlich entsprechend Beispiel 1) und 14,83 ml (0,0346 Mol) wäßrige 15%ige Titan(III)-chloridlösung in 20 ml Wasser und 100 ml Methanol werden bei 25°C unter Kühlung 12,5 ml (0,121 Mol) 30%iges Wasserstoffperoxid langsam eingetropft. Nach 1 Stunde wird das Reaktionsgemisch mehrmals mit Chloroform ausgeschüttelt. Die vereinigten organischen Phasen werden über Natriumsulfat getrocknet, filtriert und im Vakuum eingeengt. Man erhält 5,2 g (98,5% der Theorie) 4,4-Dimethyl-1-phenylsulfonyl-2-(1,2,4-triazol-1-yl)-3-pentanon vom Schmelzpunkt 135°C.

Beispiel 5

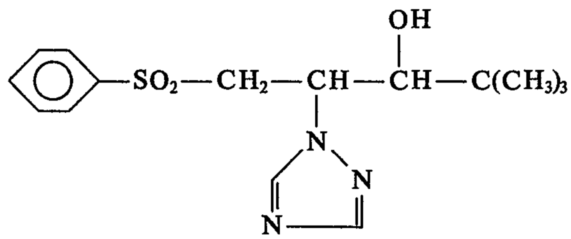

(Verfahren c)

Zu einer Suspension von 10 g (0,0328 Mol) 4,4-Dimethyl-1-phenylsulfonyl-2-(1,2,4-triazol-1-yl)-3-pentanon (Beispiel 4) und 2,42 g (0,0221 Mol) Calciumchlorid in 150 ml Isopropanol wird bei −10°C eine Lösung von 0,87 g (0,0229 Mol) Natriumborhydrid in 20 ml Wasser getropft. Nach 18 Stunden wird das Reaktionsgemisch entsprechend Beispiel 3 aufgearbeitet. Man erhält 7 g (68,6% der Theorie) 4,4-Dimethyl-1-phenylsulfonyl-2-(1,2,4-triazol-1-yl)-3-pentanol vom Schmelzpunkt 145°C.

In entsprechender Weise und gemäß den erfindungsgemäßen Verfahren werden die folgenden Verbindungen der Formel (I)

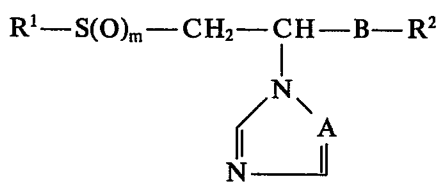

(I)

erhalten:

| Bsp. Nr. | $R^1$ | m | A | B | $R^2$ | Schmelzpunkt (°C) bzw. $n_D^{20}$ |
|---|---|---|---|---|---|---|
| 6 | 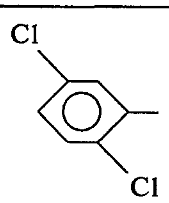 | 0 | N | CO | $C(CH_3)_3$ | 112 |
| 7 | 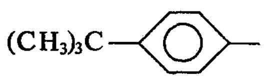 | O | N | CO | $C(CH_3)_3$ | 54 |

Fortsetzung

| Bsp. Nr. | $R^1$ | m | A | B | $R^2$ | Schmelzpunkt (°C) bzw. $n_D^{20}$ |
|---|---|---|---|---|---|---|
| 8 | 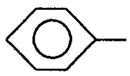 | 0 | N | CO | $C(CH_3)_3$ | zähes Öl |
| 9 | 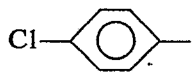 | 0 | N | CO | $-C(CH_3)_2CH_2Cl$ | 65 |
| 10 | 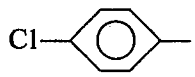 | 0 | N | CO | $-C(CH_2Cl)_2CH_3$ | 72 |
| 11 | 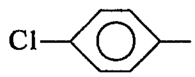 | 0 | N | CO | $-C(CH_2F)_2CH_3$ | 50 |
| 12 | 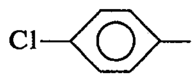 | 2 | N | CO | $-C(CH_3)_2CH_2F$ | 136 |
| 13 | 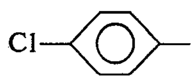 | 2 | N | CO | $-C(CH_3)_2CH_2Cl$ | 144 |
| 14 | 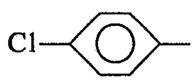 | 2 | N | CO | $-C(CH_2Cl)_2CH_3$ | zähes Öl |
| 15 | 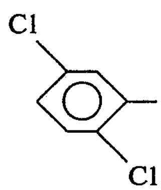 | 0 | N | CH(OH) | $C(CH_3)_3$ | 103 |
| 16 | 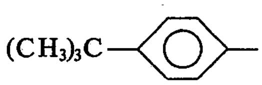 | 0 | N | $CH(CH_3)_3$ | $C(CH_3)_3$ | 106 |
| 17 | 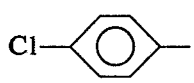 | 0 | N | CH(OH) | $-C(CH_3)_2CH_2Cl$ | 103 |
| 18 | 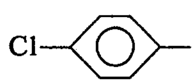 | 0 | N | CH(OH) | $-C(CH_3)_2CH_2F$ | 89 |
| 19 | 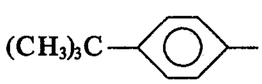 | 0 | N | CH(OH) | $-C(CH_3)_2CH_2F$ | 90 |
| 20 | 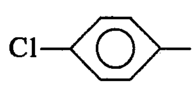 | 0 | N | CH(OH) | $-C(CH_3)_2CH_2$<br>$OCH_3$ | 68 |
| 21 | 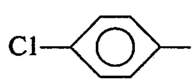 | 0 | N | CH(OH) | $-C(CH_3)_2CO$<br>$OC_2H_5$ | 70 |
| 22 | 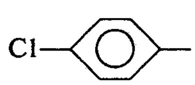 | 0 | N | CH(OH) | 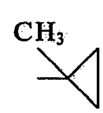 | 111 |
| 23 | 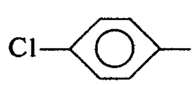 | 2 | N | CH(OH) | $C(CH_3)_3$ | 175 |
| 24 | 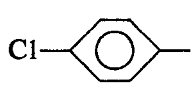 | 2 | N | CH(OH) | $-C(CH_3)_2CH_2F$ | 158 |

Fortsetzung

| Bsp. Nr. | $R^1$ | m | A | B | $R^2$ | Schmelzpunkt (°C) bzw. $n_D^{20}$ |
|---|---|---|---|---|---|---|
| 25 | 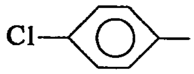 | 2 | N | CH(OH) | $-C(CH_3)_2CH_2Cl$ | 204 |
| 26 | 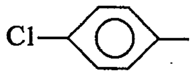 | 0 | CH | CO | $C(CH_3)_3$ | 1,5680 |
| 27 | 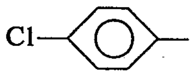 | 0 | CH | CH(OH) | $C(CH_3)_3$ | 136 |
| 28 | 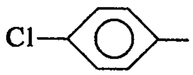 | 2 | CH | CO | $C(CH_3)_3$ | 132 |
| 29 | 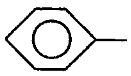 | 0 | N | CO | $-C(CH_2F)_2CH_3$ | 1,5479 |
| 30 | 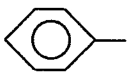 | 0 | N | CO | $-C(CH_3)_2CH_2Cl$ | 66 |
| 31 | 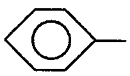 | 0 | N | CO | $-C(CH_3)_2CH_2F$ | 1,5557 |
| 32 | 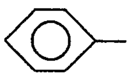 | 2 | N | CO | $-C(CH_2F_{72}CH_3$ | 116 |
| 33 | 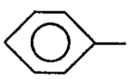 | 0 | N | CO | 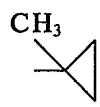 | 1,5758 |
| 34 | 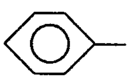 | 2 | N | CO | 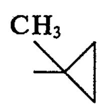 | 108 |
| 35 | 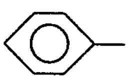 | 2 | N | CO | $-C(CH_3)_2CH_2Cl$ | 155 |
| 36 | 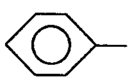 | 2 | N | CO | $-C(CH_3)_2CH_2F$ | 134 |
| 37 | 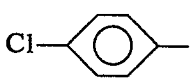 | 2 | CH | CH(OH) | $-C(CH_3)_3$ | 250 |

## Anwendungsbeispiele

In den nachfolgenden Anwendungsbeispielen werden die nachstehend angegebenen Verbindungen als Vergleichssubstanzen eingesetzt:

(A) 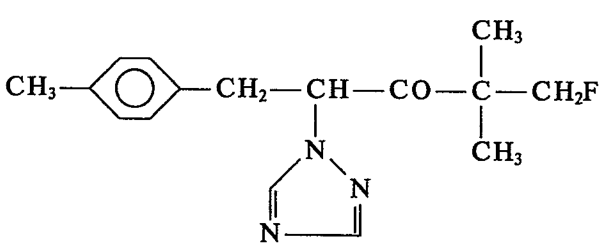

(B) 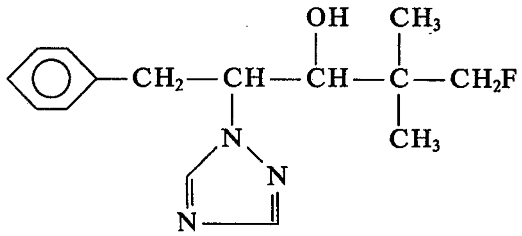

(C) 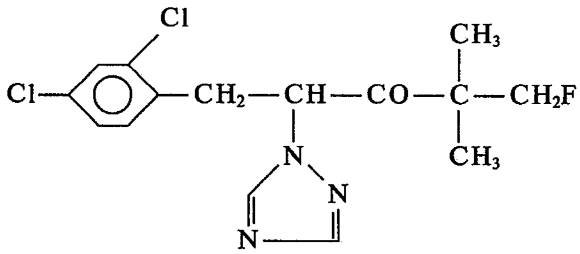

(D) 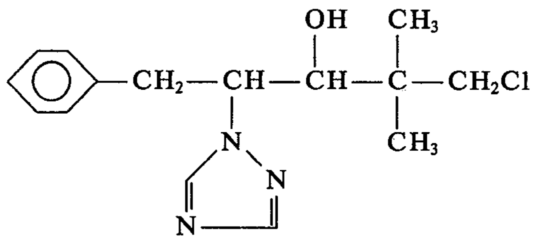

Beispiel A

Erysiphe-Test (Gerste)/protektiv

Lösungsmittel: 100 Gewichtsteile Dimethylformamid
Emulgator: 0,25 Gewichtsteile Alkylarylpolyglykoleäther

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f. sp. hordei bestäubt.

Die Pflanzen werden in einem Gewächshaus bei einer Temperatur von ca. 20°C und einer relativen Luftfeuchtigkeit von ca. 80% aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z. B. die Verbindungen gemäß folgender Herstellungsbeispiele: 6, 3, 17, 18, 21 und 12.

Beispiel B

Wuchsbeeinflussung bei Zuckerrüben

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Zuckerrüben werden im Gewächshaus bis zur vollen Ausbildung der Keimblätter angezogen. In diesem Stadium werden die Pflanzen tropfnaß mit der Wirkstoffzubereitung besprüht. Nach 14 Tagen wird der Zuwachs der Pflanzen gemessen und die Wuchsbeeinflussung in Prozent des Zuwachses der Kontrollpflanzen berechnet. Es bedeuten 0% Wuchsbeeinflussung ein Wachstum entsprechend dem der Kontrollpflanzen. Negative Werte kennzeichnen eine Wuchshemmung, positive eine Wuchsförderung gegenüber den Kontrollpflanzen.

In diesem Test zeigen z. B. die erfindungsgemäßen Herstellungsbeispiele 22, 3, 16, 15 und 7 eine stärkere Wuchsbeeinflussung als die aus dem Stand der Technik bekannten Verbindungen (B) und (C).

## Beispiel C

### Wuchshemmung bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 3 Wochen wird der Zuwachs der Pflanzen gemessen und die Wuchshemmung in Prozent des Zuwachses der Kontrolle berechnet. Es bedeuten 100% Wuchshemmung den Stillstand des Wachstums und 0% ein Wachstum entsprechend dem der Kontrollpflanzen.

In diesem Test zeigt z. B. das erfindungsgemäße Herstellungsbeispiel 3 eine stärkere Wuchshemmung als die aus dem Stand der Technik bekannten Verbindungen (A) und (C).

## Beispiel D

### Stimulierung der $CO_2$-Fixierung bei Sojabohnen

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Sojabohnenpflanzen werden im Gewächshaus bis zur vollen Entfaltung des ersten Folgeblattes angezogen. In diesem Stadium werden die Pflanzen tropfnaß mit den Wirkstoffzubereitungen besprüht. Im weiteren Verlauf des Experimentes wird die $CO_2$-Fixierung der Pflanzen mit üblichen Methoden gemessen. Die Werte werden mit denen nicht mit Wirkstoffen behandelter Kontrollpflanzen verglichen.

Weitere Versuchsdaten und die Resultate dieses Versuches gehen aus der nachfolgenden Tabelle hervor. Es bedeuten:

— Hemmung der $CO_2$-Fixierung
0 $CO_2$-Fixierung wie bei Kontrolle
\+ geringe Stimmulierung der $CO_2$-Fixierung
++ starke Stimulierung der $CO_2$-Fixierung
+++ sehr starke Stimulierung der $CO_2$-Fixierung

In diesem Test zeigen z. B. die erfindungsgemäßen Herstellungsbeispiele 21 und 20 eine stärkere Stimulierung der $CO_2$-Fixierung als die aus dem Stand der Technik bekannten Verbindungen (A), (B) und (C).

**Patentansprüche**

1. Azolyl-thioether-Derivate der Formel

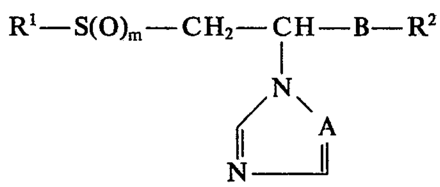 (I)

in welcher

A für ein Stickstoffatom oder die CH-Gruppe steht,

B für die CO- oder CH(OH)-Gruppe steht,
m für die Zahlen 0 oder 2 steht,
$R^1$ für gegebenenfalls einfach oder mehrfach, gleich oder verschieden substituiertes Phenyl steht, wobei als Substituenten Halogen, Alkyl mit 1 bis 4 Kohlenstoffatomen, Halogenalkyl, Halogenalkoxy und Halogenalkylthio mit jeweils 1 bis 2 Kohlenstoff- und 1 bis 5 gleichen oder verschiedenen Halogenatomen, Nitro, Cyano und gegebenenfalls durch Halogen oder Alkyl mit 1 bis 2 Kohlenstoffatomen substituiertes Phenyl zu nennen sind,
$R^2$ für gegebenenfalls durch Alkyl mit 1 bis 4 Kohlenstoffatomen substituiertes Cycloalkyl mit 3 bis 7 Kohlenstoffatomen, sowie für die Gruppierungen

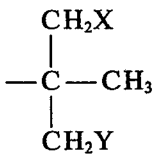 und 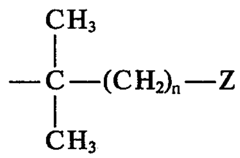

steht, wobei

X für Wasserstoff, Fluor oder Chlor steht,
Y für Wasserstoff, Fluor oder Chlor steht,
Z für Alkyl mit 1 bis 4 Kohlenstoffatomen, für jeweils gegebenenfalls substituiertes Phenyl, Phenoxy, Phenylthio, Benzyloxy oder Benzylthio steht, wobei als Phenylsubstituenten die bei $R^1$ erwähnten Phenylsubstituenten zu nennen sind, ferner für Alkoxy und Alkylthio mit jeweils 1 bis 4 Kohlenstoffatomen, für Halogenalkoxy und Halogenalkylthio mit jeweils 1 bis 2 Kohlenstoff- und 1 bis 5 gleichen oder verschiedenen Halogenatomen steht, sowie für Alkoxycarbonyl mit 1 bis 4 Kohlenstoffatomen im Alkylteil und für Cyano steht, und
n für die Zahlen 0 oder 1 steht,

sowie deren Säureadditions-Salze und Metallsalz-Komplexe.

2. Azolyl-thioether-Derivate der Formel (I) in Anspruch 1, wobei

$R^1$ für gegebenenfalls einfach bis dreifach, gleich oder verschieden substituiertes Phenyl steht, wobei als Substituenten Fluor, Chlor, Methyl, Ethyl, Isopropyl, tert.-Butyl, Trifluormethyl, Trifluormethoxy, Trifluormethylthio, Nitro, Cyano, sowie gegebenenfalls durch Fluor, Chlor und/oder Methyl substituiertes Phenyl zu nennen sind,
$R^2$ für gegebenenfalls durch Methyl, Ethyl oder Propyl substituiertes Cyclopropyl, Cyclopentyl und Cyclohexyl steht, sowie für die Gruppierungen

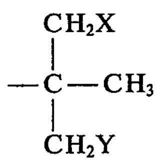 und 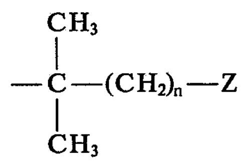

steht, wobei

X für Wasserstoff, Fluor oder Chlor steht,
Y für Wasserstoff, Fluor oder Chlor steht,
Z für Alkyl, Alkoxy und Alkylthio mit jeweils 1 bis 4 Kohlenstoffatomen, für Trifluormethoxy und Trifluormethylthio, für Methoxy-, Ethoxy- und Isopropoxycarbonyl sowie für Cyano steht, ferner für gegebenenfalls einfach bis dreifach substituiertes Phenyl, Phenoxy oder Phenylthio steht, wobei als Substituenten die bei $R^1$ bereits genannten Phenylsubstituenten infrage kommen, und
A, B, m und n die in Anspruch 1 angegebenen Bedeutungen besitzen.

3. Verfahren zur Herstellung von Azolyl-thioether-Derivaten der Formel

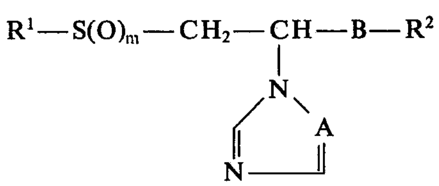 (I)

in welcher

A für ein Stickstoffatom oder die CH-Gruppe steht,
B für die CO- oder CH(OH)-Gruppe steht,
m für die Zahlen 0 oder 2 steht,
$R^1$ für gegebenenfalls einfach oder mehrfach, gleich oder verschieden substituiertes Phenyl steht, wobei als Substituenten Halogen, Alkyl mit 1 bis 4 Kohlenstoffatomen, Halogenalkyl, Halogenalkoxy und Halogenalkylthio mit jeweils 1 bis 2 Kohlenstoff- und 1 bis 5 gleichen oder verschiedenen Halogenatomen, Nitro, Cyano und gegebenenfalls durch Halogen oder Alkyl mit 1 bis 2 Kohlenstoffatomen substituiertes Phenyl zu nennen sind,
$R^2$ für gegebenenfalls durch Alkyl mit 1 bis 4 Kohlenstoffatomen substituiertes Cycloalkyl mit 3 bis 7 Kohlenstoffatomen, sowie für die Gruppierungen

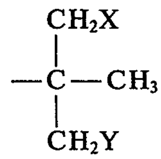 und 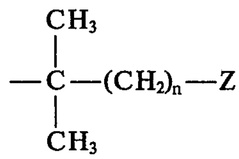

steht, wobei

X für Wasserstoff, Fluor oder Chlor steht,
Y für Wasserstoff, Fluor oder Chlor steht,
Z für Alkyl mit 1 bis 4 Kohlenstoffatomen, für jeweils gegebenenfalls substituiertes Phenyl, Phenoxy, Phenylthio, Benzyloxy oder Benzylthio steht, wobei als Phenylsubstituenten die bei $R^1$ erwähnten Phenylsubstituenten zu nennen sind, ferner für Alkoxy und Alkylthio mit jeweils 1 bis 4 Kohlenstoffatomen, für Halogenalkoxy und Halogenalkylthio mit jeweils 1 bis 2 Kohlenstoff- und 1 bis 5 gleichen oder verschiedenen Halogenatomen steht, sowie für Alkoxycarbonyl mit 1 bis 4 Kohlenstoffatomen im Alkylteil und für Cyano steht, und
n für die Zahlen 0 oder 1 steht,

dadurch gekennzeichnet, daß man
a) Azolylketone der Formel

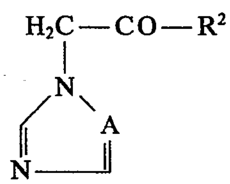 (II)

in welcher
A und $R^2$ die oben angegebene Bedeutung haben,
mit Formaldehyd oder Formaldehyd abgebenden Stoffen und einem Thio-Derivat der Formel

$$R^1—S—H \quad \text{(III)}$$

in welcher
$R^1$ die oben angegebene Bedeutung hat,
in Gegenwart eines Verdünnungsmittels und in Gegenwart eines Katalysators umsetzt; und gegebenenfalls
b) die so erhaltenen Verbindungen der Formel

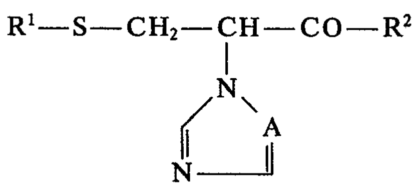 (Ia)

in welcher
A, $R^1$ und $R^2$ die oben angegebene Bedeutung haben,
nach bekannten Methoden in üblicher Weise zu den entsprechenden $SO_2$-Derivaten oxidiert, und gegebenenfalls
c) die nach den Verfahren (a) und (b) erhaltenen Verbindungen der Formel

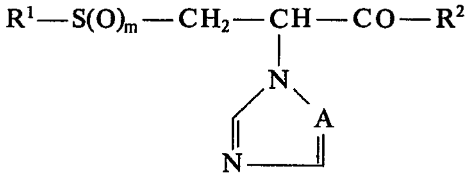

(Ib)

in welcher
A, $R^1$, $R^2$ und m die oben angegebene Bedeutung haben,
nach bekannten Methoden in üblicher Weise zu den entsprechenden CH(OH)-Derivaten reduziert; und gegebenenfalls noch zur Herstellung von Säureadditions-Salzen und Metallsalz-Komplexen anschließend an die nach den Verfahren (a), (b) und (c) erhaltenen Verbindungen der Formel (I) in üblicher Weise eine Säure oder ein Metallsalz addiert.

4. Fungizide und das Wachstum von Pflanzen regulierende Mittel, gekennzeichnet durch einen Gehalt an mindestens einem Azolyl-thioether-Derivat der Formel (I).

5. Verwendung von Azolyl-thioether-Derivaten der Formel (I) zur Bekämpfung von Pilzen und zur Regulierung des Pflanzenwachstums.

**Claims**

1. Azolyl-thioether derivatives of the formula

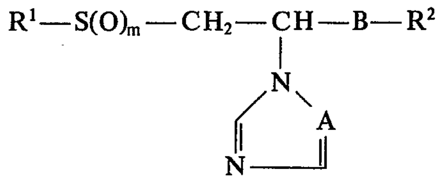

(I)

in which

A represents a nitrogen atom of the CH group,
B represents the CO or CH(OH) group,
m represents the numbers 0 or 2,
$R^1$ represents phenyl which is optionally mono- or polysubstituted by identical or different substituents which may be named as follwos: halogen, alkyl with 1 to 4 carbon atoms, halogenoalkyl, halogenoalkoxy and halogenoalkylthio each with 1 to 2 carbon and 1 to 5 identical or different halogen atoms, nitro, cyano and phenyl which is optionally substituted by halogen or alkyl with 1 to 2 carbon atoms,
$R^2$ represents cycloalkyl which has 3 to 7 carbon atoms and is optionally substituted by alkyl with 1 to 4 carbon atoms, and the groupings

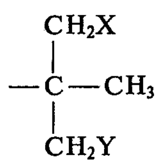 and 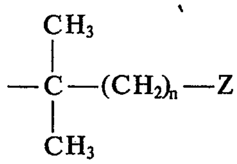

wherein

X represents hydrogen, fluorine or chlorine,
Y represents hydrogen, fluorine or chlorine,
Z represents alkyl with 1 to 4 carbon atoms, phenyl, phenoxy, phenylthio, benzyloxy or benzylthio, each of which is optionally substituted, the phenyl substituents which may be mentioned being those phenyl substituents mentioned under $R^1$, and also represents alkoxy and alkylthio each with 1 to 4 carbon atoms, halogenoalkoxy and halogenoalkylthio each with 1 to 2 carbon and 1 to 5 identical or different halogen atoms, and also represents alkoxycarbonyl with 1 to 4 carbon atoms in the alkyl part and cyano, and
n represents the numbers 0 or 1,

and acid addition salts and metal complexes thereof.

2. Azolyl-thioether derivates of the formula (1) in Claim 1, wherein

$R^1$ represents phenyl which is optionally mono- to trisubstitutes by identical or different substituents which may be named as follows: fluorine, chlorine, methyl, ethyl, isopropyl, tert.-butyl, trifluoromethyl, trifluoromethoxy, trifluoromethylthio, nitro, cyano and phenyl which is optionally substituted by fluorine, chlorine and/or methyl,

$R^2$ represents cyclopropyl, cyclopentyl and cyclohexyl which are optionally substituted by methyl, ethyl or propyl, and represents the groupings

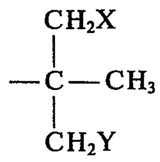 and 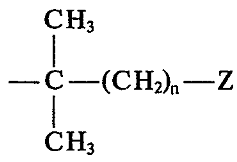

wherein

X represents hydroden, fluorine or chlorine,
Y represents hydrogen, fluorine or chlorine,
Z represents alkyl, alkoxy and alkylthio each with 1 to 4 carbon atoms, trifluoromethoxy and trifluoromethylthio, methoxy-, ethoxy- and isopropoxycarbonyl and cyano, and also represents optionally mono- to trisubstituted phenyl, phenoxy or phenylthio, possible substituents being the phenyl substituents already mentioned under $R^1$, and
A, B, m und n have the meanings given in Claim 1.

3. Process for the preparation of azolyl-thioether derivatives of the formula

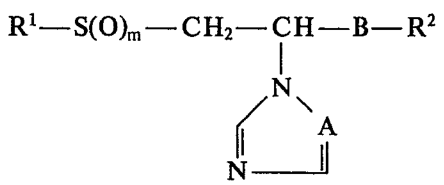 (I)

in which

A represents a nitrogen atom or the CH group,
B represents the CO or CH(OH) group,
m represents the numbers 0 or 2,
$R^1$ represents phenyl which is optionally mono- or polysubstituted by identical or different substituents which may be named as follows: halogen, alkyl with 1 to 4 carbon atoms, halogenoalkyl, halogenoalkoxy and halogenoalkylthio each with 1 to 2 carbon and 1 to 5 identical or different halogen atoms, nitro, cyano and phenyl which is optionally substituted by halogen or alkyl with 1 to 2 carbon atoms,
$R^2$ represents cycloalkyl which has 3 to 7 carbon atoms and is optionally substituted by alkyl with 1 to 4 carbon atoms, and the groupings

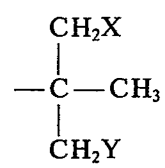 and 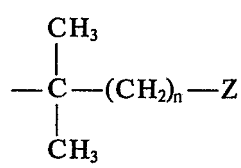

wherein

X represents hydrogen, fluorine or chlorine,
Y represents hydrogen, fluorine or chlorine,
Z represents alkyl with 1 to 4 carbon atoms, phenyl, phenoxy, phenylthio, benzyloxy or benzylthio, each of which is optionally substituted, the phenyl substituents which may be mentioned being those phenyl substituents mentioned under $R^1$, and also represents alkoxy and alkylthio each with 1 to 4 carbon atoms, halogenoalkoxy and halogenoalkylthio each with 1 to 2 carbon and 1 to 5 identical or different halogen atoms, and also represents alkoxycarbonyl with 1 to 4 carbon atoms in the alkyl part and cyano, and
n represents the numbers 0 or 1,

characterised in that

a) azolyl ketones of the formula

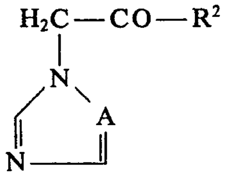

(II)

in which
A and $R^2$ have the abovementioned meaning,
are reacted with formaldehyde of formaldehyde-donating substances and a thio derivative of the formula

$$R^1—S—H \qquad (III)$$

in which
$R^1$ has the abovementioned meaning,
in the presence of a diluent and in the presence of a catalyst; and, if desired,

b) the compounds thus obtained, of the formula

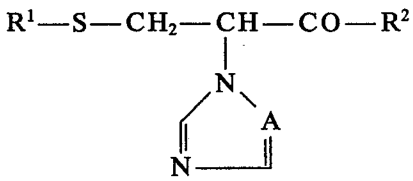

(Ia)

in which
A, $R^1$ and $R^2$ have the abovementioned meaning,
are oxidised to the corresponding $SO_2$ derivatives by known methods in a customary manner, and, if desired,

c) the compounds obtained by processes (a) and (b), of the formula

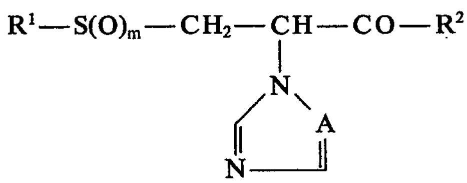

(Ib)

in which
A, $R^1$, $R^2$ and m have the abovementioned meaning,
are reducted to the corresponding CH(OH) derivatives by known methods in a customary manner; and, if desired, to prepare acid addition salts and metal salt complexes, an acid or a metal salt is then furthermore added on, in a customary manner, to the compounds of the formula (I) obtained by processes (a), (b) and (c).

4. Fungicides and agents regulating the growth of plants, characterised by a content of a least one azolyl-thioether derivative of the formula (I).

5. Use of azolyl-thioether derivatives of the formula (I) for combating fungi and for regulating plant growth.

**Revendications**

1. Dérivés d'azolyl-thioéthers de formule:

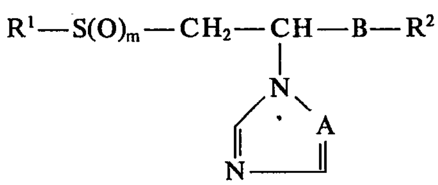

(I)

dans laquelle

A représente un atome d'azote ou le groupe CH,
B représente le groupe CO ou le groupe CH(OH),
m représente les nombres 0 ou 2,
$R^1$ représente un groupe phényle éventuellement substitué une ou plusieurs fois de manière identique ou différente et, parmi les substituants, il convient de mentionner les atomes d'halogènes, les groupes alkyle contenant 1 à 4 atomes de carbone, les groupes halogénalkyle, les groupes halogénalcoxy et les groupes halogénalkylthio contenant chacun 1 ou 2 atomes de carbone et 1 à 5 atomes d'halogènes identiques ou différents, le groupe nitro, le groupe cyano et le groupe phényle éventuellement substitué par une atome d'halogène ou par un groupe alkyle contenant 1 ou 2 atomes de carbone,
$R^2$ représente un groupe cycloalkyle contenant 3 à 7 atomes de carbone et éventuellement substitué par un groupe alkyle contenant 1 à 4 atomes de carbone, ainsi que les groupements:

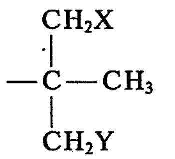 et 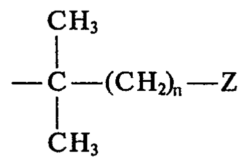

X représentant un atome d'hydrogène, un atome de fluor, ou un atome de chlore,
Y représentant un atome d'hydrogène, un atome de fluor, ou un atome de chlore,
Z représentant un groupe alkyle contenant 1 à 4 atomes de carbone, un groupe benzylthio, un groupe benzyloxy, un groupe phénylthio, un groupe phénoxy ou un groupe phényle éventuellement substitué chaque fois en montionnant, comme substituants du groupe phényle, les substituants du groupe phényle cités pour $R^1$, ainsi qu'un groupe alcoxy et un groupe alkylthio contenant chacun 1 à 4 atomes de carbone, un groupe halogénalcoxy et un groupe halogénalkylthio contenant chacun 1 ou 2 atomes de carbone et 1 à 5 atomes d'halogènes identiques ou différents, ainsi qu'un groupe alcoxycarbonyle contenant 1 à 4 atomes de carbone dans la fraction alkyle, de même qu'un groupe cyano, et
n représentant les nombres 0 ou 1,

ainsi que leurs sels d'addition d'acide et leur complexes métalliques.

2. Dérivés d'azolyl-thioéthers de formule (I) selon la revendication 1, dans lesquels

$R^1$ représente un groupe phényle éventuellement substitué une à trois fois de manière identique ou différente en mentionnant, comme substituants, l'atome de fluor, l'atome de chlore, le groupe méthyle, le groupe éthyle, le groupe isopropyle, le groupe tert-butyle, le groupe trifluorométhyle, le groupe trifluorométhoxy, le groupe trifluorométhylthio, le groupe nitro, le groupe cyano, ainsi que le groupe phényle éventuellement substitué par l'atome de fluor, par l'atome de chlore et/ou par le groupe méthyle,
$R^2$ représente le groupe cyclopropyle, le groupe cyclopentyle et le groupe cyclohexyle éventuellement substitué par le groupe méthyle, par le groupe éthyle ou par le groupe propyle, ainsi que les groupements

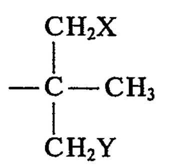 et 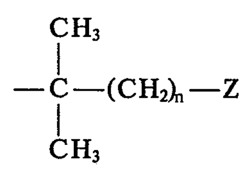

X représentant un atome d'hydrogène, un atome de fluor ou un atome de chlore,
Y représentant un atome d'hydrogène, un atome de fluor ou un atome de chlore,
Z représentant un groupe alkyle, un groupe alcoxy et un groupe alkylthio contenant chacun 1 à 4 atomes de carbone, le groupe trifluorométhoxy et le groupe trifluorométhylthio, le groupe méthoxycarbonyle, le groupe éthoxycarbonyle et le groupe isopropoxycarbonyle, de même que le groupe cyano, ainsi que le groupe phénylthio, le groupe phénoxy ou le groupe phényle éventuellement substitué une à trois fois en envisageant, comme substituants, les substituants du groupe phényle qui ont déjà été mentionnés pour $R^1$, et
A, B, m et n ayant les significations indiquées dans la revendication 1.

3. Procédé de préparation de dérivés d'azolyl-thioéthers de formule:

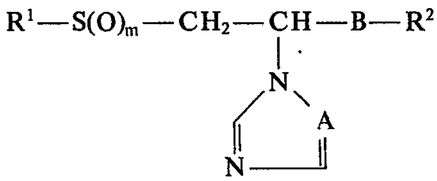

(I)

dans laquelle

A représente un atome d'azote ou le groupe CH,
B représente le groupe CO ou le groupe CH(OH),
m représente les nombres 0 ou 2,
$R^1$ représente un groupe phényle éventuellement substitué une ou plusieurs fois de manière identique ou différente et, parmi les substituants, il convient de mentionner les atomes d'halogènes, les groupes alkyle contenant 1 à 4 atomes de carbone, les groupes halogénalkyle, les groupes halogénalcoxy et les groupes halogénalkylthio contenant chacun 1 ou 2 atomes de carbone et 1 à 5 atomes d'halogènes identiques ou différents, le groupe nitro, le groupe cyano et le groupe phényle éventuellement substitué par un atome d'halogène ou par un groupe alkyle contenant 1 ou 2 atomes de carbone,
$R^2$ représente un groupe cycloalkyle contenant 3 à 7 atomes de carbone et éventuellement substitué par un groupe alkyle contenant 1 à 4 atomes de carbone, ainsi que les groupements:

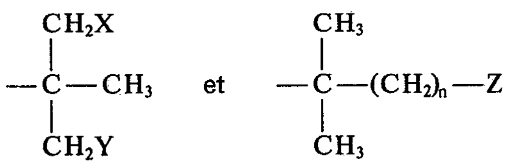

X représentant un atome d'hydrogène, un atome de fluor, ou un atome de chlore,
Y représentant un atome d'hydrogène, un atome de fluor, ou un atome de chlore,
Z représentant un groupe alkyle contenant 1 à 4 atomes de carbone, un groupe benzylthio, un groupe benzyloxy, un groupe phénylthio, un groupe phénoxy ou un groupe phényle éventuellement substitué chaque fois en mentionnant, comme substituants du groupe phényle, les substituants du groupe phényle cités pour $R^1$, ainsi qu'un groupe alcoxy et un groupe alkylthio contenant chacun 1 à 4 atomes de carbone, un groupe halogénalcoxy et un groupe halogénalkylthio contenant chacun 1 ou 2 atomes de carbone et 1 à 5 atomes d'halogènes identiques ou différents, ainsi qu'un groupe alcoxycarbonyle contenant 1 à 4 atomes de carbone dans la fraction alkyle, de même qu'un groupe cyano, et
n représentant les nombres 0 ou 1,

caracterisé en ce que:
a) on fait réagir des azolyl-cétones de formule:

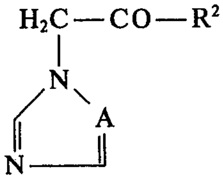

(II)

dans laquelle
A et $R^2$ ont les significations indiquées ci-dessus, avec du formaldéhyde ou des substances fournissant du formaldéhyde et un dérivé thio de formule:

$$R^1—S—H \qquad (III)$$

dans laquelle
$R^1$ a la signification indiquée ci-dessus,
en présence d'un diluant, ainsi qu'en présence d'un catalyseur; et éventuellement
b) on oxyde les composés ainsi obtenus de formule:

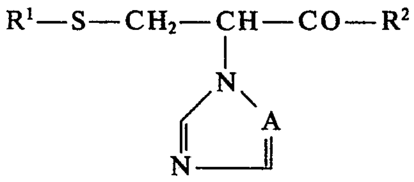

(Ia)

dans laquelle
A, $R^1$ et $R^2$ ont les significations indiquées ci-dessus,
selon des méthodes connues et de la manière habituelle, pour obtenir les dérivés $SO_2$ correspondants, et éventuellement

c) on réduit les composés obtenus selon les procédés (a) et (b) et répondant à la formule:

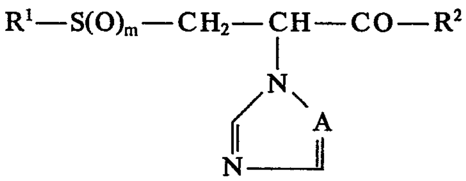

(Ib)

dans laquelle
A, $R^1$, $R^2$ et m ont les significations indiquées ci-dessus,
selon des méthodes connues et de la manière habituelle, en dérivés CH(OH) correspondants; et éventuellement encore, pour préparer des sels d'addition d'acide et des complexes métalliques, aux composés de formule (I) obtenus selon les procédés (a), (b) et (c), on ajoute ensuite, de la manière habituelle, un acide ou un sel métallique.

4. Fongicides et agents régulateurs de la croissance des plantes, caractérisés en ce qu'ils contiennent au moins un dérivé d'azolyl-thioéther de formule (I).

5. Utilisation de dérivés d'azolyl-thioéthers de formule (I) pour combattre les champignons et pour régler la croissance des plantes.